(19) Europäisches Patentamt
European Patent Office
Office européen des brevets

(11) Veröffentlichungsnummer: **0 065 136**
**B1**

(12) **EUROPÄISCHE PATENTSCHRIFT**

(45) Veröffentlichungstag der Patentschrift: **14.11.84**

(51) Int. Cl.³: **C 08 F 4/04**, C 08 F 2/24, C 07 C 107/00

(21) Anmeldenummer: **82103591.2**

(22) Anmeldetag: **28.04.82**

(54) **Oberflächenaktive Azoverbindungen und ihre Verwendung.**

(30) Priorität: **09.05.81 DE 3118372**

(43) Veröffentlichungstag der Anmeldung: **24.11.82 Patentblatt 82/47**

(45) Bekanntmachung des Hinweises auf die Patenterteilung: **14.11.84 Patentblatt 84/46**

(84) Benannte Vertragsstaaten: **DE FR GB IT NL**

(56) Entgegenhaltungen:
**EP-A-0 009 186**
**DE-B-1 111 395**
**GB-A-1 198 782**

(73) Patentinhaber: **BAYER AG, Konzernverwaltung RP Patentabteilung, D-5090 Leverkusen 1 Bayerwerk (DE)**

(72) Erfinder: **Schmidt, Adolf, Dr., Roggendorfstrasse 67, D-5000 Köln 80 (DE)**
Erfinder: **Roos, Ernst, Dr., Am Gensgarten 6, D-5068 Odenthal (DE)**

EP 0 065 136 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft Azo- und Paraffinsulfonatgruppen enthaltende, wasserlösliche, oberflächenaktive Substanzen, die als emulgierend wirkende Initiatoren zur Herstellung von elektrolytarmen Dispersionen mit einer geringen Tendenz zur Schaumbildung auf Basis olefinisch ungesättigter Monomerer eingesetzt werden können.

Es ist bekannt, Azodiisobuttersäureamidin und davon abgeleitete Derivate, z.B. N-Alkylierungs- und N-Oxalkylierungsprodukte in Form der Salze oder freien Basen als wasserlösliche Initiatoren bei der Emulsionspolymerisation olefinisch ungesättigter Monomerer einzusetzen (vgl. US-PS Nrn. 2599300, 2810702, DE-OS Nr. 2841045).

Es ist ferner bekannt, Alkylsulfonate als anionenaktive Emulgatoren für die Emulsionspolymerisation olefinisch ungesättigter Monomerer zu verwenden (vgl. „Methoden der Organischen Chemie", Houben-Weyl, Bd. 14/1 [1961] Seiten 196-198).

Die in Gegenwart der vorgenannten Azoinitiatoren und Emulgatoren herstellbaren Dispersionen neigen jedoch zur Schaumbildung. Aufgabe der vorliegenden Anmeldung war daher die Bereitstellung eines Initiator-Emulgator-Systems, das die Herstellung von Dispersionen mit möglichst geringer Tendenz zur Schaumbildung erlaubt. Gleichzeitig sollten die Dispersionen einen möglichst geringen Elektrolytgehalt aufweisen.

Die Aufgabe wurde dadurch gelöst, dass neue oberflächenaktive Azoverbindungen mit eingebauten Paraffinsulfonatgruppen zur Verfügung gestellt wurden, die gleichzeitig emulgierende und aktivierende Eigenschaften besitzen. Diese neuen Substanzen wirken zunächst als Emulgatoren, zerfallen während der Polymerisation, wobei die Katalysatorbruchstücke mit den Paraffinsulfonatgruppen in das Polymerisat eingebaut werden und die Latexteilchen vor Koagulation schützen.

Gegenstand der Erfindung sind somit oberflächenaktive Azoverbindungen, erhältlich durch Umsetzung von Azoverbindungen der allgemeinen Formel I

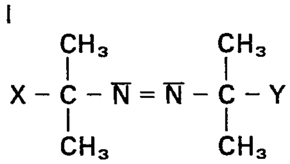 (I)

worin X und Y gleich oder verschieden sein können und wobei X und Y

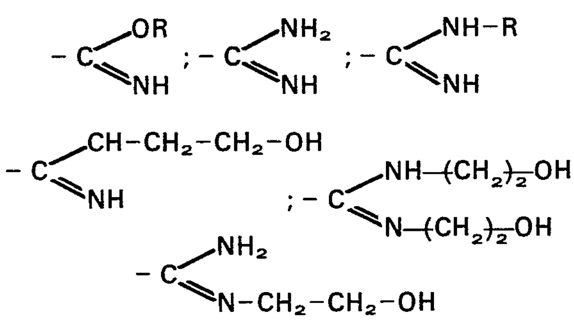

und R = $C_1$ bis $C_4$-Alkyl bedeuten,

mit mindestens einem $C_{10}$-$C_{18}$-Paraffindisulfonsäuredihalogenid unter Halogenwasserstoffabspaltung in wässerigem Medium oder einem gegenüber den Reaktanten unter den Prozessbedingungen inerten Lösungsmittel oder Lösungsmittelgemisch, worin die Reaktanten zumindest teilweise löslich sind, bei Temperaturen von 0 bis 30°C in Gegenwart einer dem verseifbaren Halogen im wesentlichen äquivalenten Menge einer Base.

Bevorzugte Reste X und Y sind

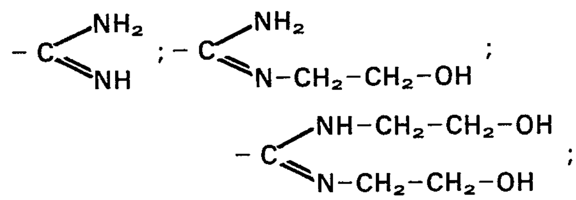

insbesondere 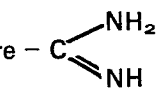

Die Azoverbindungen der allgemeinen Formel I sind bekannt, ebenso die erfindungsgemäss einzusetzenden $C_{10}$-$C_{18}$-Paraffindisulfonsäuredihalogenide. Bevorzugt werden $C_{10}$-$C_{18}$-Paraffindisulfonsäuredichloride, insbesondere $C_{14}$-$C_{16}$-Paraffindisulfonsäuredichloride. Vorzugsweise werden Gemische der Paraffindisulfonsäuredichloride eingesetzt (zur Herstellung vgl. F. Asinger, „Die petrolchemische Industrie", Teil I, S. 731 bis 738, Akademie-Verlag Berlin [1971]). Die Paraffindisulfonsäuredichloride können von ihrer Herstellung her noch geringe Mengen Halogen (bis ca. 5 Gew.-%) in der Paraffinkette enthalten. Die angeführten Zahlen der Kohlenstoffatome der Paraffindisulfonsäuredihalogenide sind Durchschnittswerte.

Die Reaktion aromatischer Monosulfonsäurechloride mit monofunktionellen Iminoethern oder monofunktionellen Amidinen ist an sich bekannt.

Bei der Umsetzung von Sulfonylchloriden mit Iminoetherbasen entstehen neben den Sulfonyliminoethern noch Sulfonylamide. Bei der Reaktion von Sulfonylchloriden mit Amidinen entstehen tautomere Sulfonylamidine unterschiedlicher Stabilität, welche in Gegenwart von Wasser unterschiedliche Verseifungsprodukte ergeben können (vgl. hierzu H.J. Barber, „J. Chem. Soc." [1943], 101-104).

Zur Herstellung der oberflächenaktiven Azoverbindungen werden auf 1 mol Azoverbindung der allgemeinen Formel I oder deren Salze bevorzugt 1 bis 2 mol, insbesondere etwa 2 mol, der Paraffindisulfonsäuredihalogenide eingesetzt.

Die Umsetzung erfolgt bei Temperaturen unterhalb der Zersetzungstemperatur der Azoverbindungen, vorzugsweise bei 0 bis 30, insbesondere 5 bis 20°C.

Als Basen werden Alkalimetall- oder Erdalkalimetallhydroxide bzw. Ammoniak in Form ihrer wässerigen Lösungen verwendet, vorzugsweise Alkalimetallhydroxide, wie Natrium- oder Kaliumhydroxid, und zwar in einer dem bei 0 bis 30°C

verseifbaren Halogen der Disulfonsäuredihalogenidgruppen im wesentlichen äquivalenten Menge. Werden die Azoverbindungen der allgemeinen Formel I in Form ihrer Salze eingesetzt, so wird die Menge an Basen so erhöht, dass die Azoverbindungen der Formel I als Basen vorliegen. Das gegebenenfalls in der Paraffinkette der Paraffindisulfonsäuredihalogenide enthaltene Halogen wird unter den gegebenen Reaktionsbedingungen durch die Basen praktisch nicht verseift.

Als Reaktionsmedium dient vorzugsweise Wasser. Es können jedoch auch Lösungsmittel wie Pyridin, Tetrahydrofuran, Dimethylformamid oder Ketone wie Aceton oder Gemische der Lösungsmittel als Reaktionsmedien verwendet werden.

Die Umsetzung der Azoverbindungen der allgemeinen Formel I bzw. deren Salze mit den Paraffindisulfonsäuredihalogeniden kann vorteilhaft so durchgeführt werden, dass die Azoverbindung, gelöst im Reaktionsmedium, vorzugsweise Wasser, vorgelegt und das Paraffindisulfonsäuredihalogenid und die Base getrennt, aber gleichzeitig bei einer im wesentlichen konstanten Temperatur innerhalb des Temperaturbereiches von 0 bis 30°C zudosiert werden. Während der Umsetzung sollte der pH-Wert des Reaktionsgemisches im Bereich von 7 bis 10 gehalten werden. Die Reaktion verläuft exotherm und wird durch geeignete Kühlmassnahmen auf der gewählten Temperatur gehalten. Am Ende der Umsetzung resultiert ein neutrales bis schwach alkalisches (pH: 7-8,5) Reaktionsmedium.

Die Aufarbeitung des Reaktionsgemisches kann durch schonendes Eindampfen bis zur Trockene erfolgen. Der Rückstand wird dann mit Methanol oder Ethanol versetzt, wobei die Sulfonatgruppen enthaltenden Azoverbindungen in Lösung gehen und von den Salzen leicht abgetrennt werden kann. Nach Abdampfen des Alkohols erhält man die salzfreien, Sulfonatgruppen enthaltenden Azoverbindungen.

Eine andere Möglichkeit der Aufarbeitung besteht in der Extraktion des wässerigen Reaktionsgemisches mit geeigneten mit Wasser nicht mischbaren Lösungsmitteln wie niedrigsiedenden Ethern.

Die erfindungsgemäss erhaltenen, oberflächenaktiven Azoverbindungen stellen wasserlösliche, wachsartige bis sirupöse Substanzen dar, die mittlere Molgewichte $\overline{M}_n$ (Zahlenmittel) von 0,8 bis $3{,}0 \times 10^3$, bestimmt durch Membranosmometrie unter Berücksichtigung der Stavermann-Koeffizienten besitzen. Wie durch Dünnschichtchromatographie und IR-Spektren und Azogruppenbestimmung sichergestellt, enthalten sie Azogruppen und Sulfonatgruppen, sind jedoch praktisch frei von Paraffindisulfonaten, d.h. von Verseifungsprodukten der Paraffindisulfonsäuredihalogenide. Eine genaue Strukturformel lässt sich für die emulgierend wirkenden Azoinitiatoren nicht angeben, da es sich um Stoffgemische handelt. Die erfindungsgemässen Azoverbindungen sind Initiatoren und besitzen gute Emulgiereigenschaften.

Gegenstand der Erfindung ist daher auch ihre Verwendung bei der Emulsions- und Dispersionspolymerisation eines oder mehrerer olefinisch ungesättigter Monomerer. Die oberflächenaktiven Azoverbindungen werden in Salzform, vorzugsweise als Alkalimetallsalze, in Mengen von 1,5 bis 5, vorzugsweise 2 bis 3 Gew.-%, bezogen auf Monomere, eingesetzt.

Die erfindungsgemässen Azoverbindungen können bei der Polymerisation wie übliche Emulgatoren gehandhabt werden, d.h. sie können entweder vollständig vorgelegt werden oder sie können im Rahmen eines Zulaufverfahrens zum Teil vorgelegt und zum Teil während der Emulsionspolymerisation nachdosiert werden.

Entsprechend der Zerfallskinetik der Azoemulgatoren wird die Polymerisation bei Temperaturen zwischen 35 bis 90°C durchgeführt. Der bevorzugte Temperaturbereich beträgt 45 bis 75°C.

An polymerisierbaren Monomeren kommen alle überlicherweise mit Azodiisobuttersäurenitril polymerisierbaren, olefinisch ungesättigten Monomeren in Betracht, beispielsweise Styrol, α-Methylstyrol, Butadien, Acrylsäureester mit 1 bis 8 C-Atomen in der Alkoholkomponente, Methacrylsäureester mit 1 bis 8 C-Atomen in der Alkoholkomponente, Acrylnitril, Methacrylnitril, Vinylchlorid, Vinylacetat, Ethylen, Isopren, Chloropren usw.

Neben den genannten Monomeren können zusätzlich, in geringerem Anteil, wasserlösliche Verbindungen wie Methacrylsäure, Acrylsäure, Maleinsäurehalbester, Itaconsäure und Itaconsäurehalbester, Acrylamid, Methacrylamid usw. einpolymerisiert werden. Ferner können noch funktionelle Gruppen z.B. OH- oder Epoxigruppen tragende Comonomere mitverwendet werden, wie β-Hydroxiethyl(meth)acrylat, β-Hydroxipropyl(meth)acrylat, Glycidyl(meth)acrylat und N-Methylol- bzw. N-Methylolalkylether des (Meth)acrylsäureamids.

Prinzipiell ist es natürlich möglich, die erfindungsgemässen emulgierend wirkenden Azoverbindungen im Gemisch mit üblichen anionischen oder nichtionischen Emulgatoren einzusetzen.

Die vorteilhaften und überraschenden Eigenschaften der erfindungsgemässen Azoverbindungen gehen aus den Beispielen und Vergleichsversuchen dieser Anmeldung hervor.

Die in den Beispielen und Vergleichsversuchen angegebenen Prozente und Teile beziehen sich auf das Gewicht, sofern nicht anders vermerkt.

*Beispiel 1:*

*(Azoemulgator A, erfindungsgemäss)*

90 g Azodiisobuttersäureamidin (0,45 mol) der Formel I

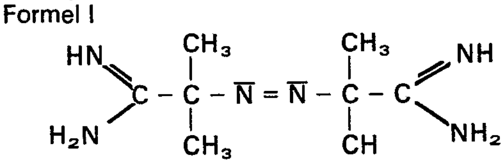

(I)

werden in 400 ml eiskaltem, entionisiertem Wasser unter gutem Rühren gelöst. Sofort nach Auflösung des Amidins werden unter Aussenkühlung mit Eis (Innentemperatur ca. +10°C) 372 g eines Gemisches sulfochlorierter Paraffine mit einem Sulfochlorgehalt von ca. 17%, einem Gesamtchlorgehalt von ca. 20,5% und einer mittleren C-Zahl von 15 gleichzeitig mit 217,8 g 50%iger Natronlauge im Verlauf von 30 min zur Lösung des vorgelegten Azodiisobuttersäureamidins bei einem pH-Wert von 7 bis 10 zugetropft.

Nach der Zugabe des Paraffinsulfochloridgemisches und der Natronlauge wird bei 15 bis 20° C so lange nachgerührt, bis das Reaktionsgemisch einen pH-Wert von ca. 8 aufweist.

Aus dem Reaktionsgemisch lässt sich das Wasser am Rotationsverdampfer im Vakuum (ca. 1 mbar) ohne Schaumbildung entfernen. Der pastöse Rückstand wird mit Methanol verrührt, wobei die Azoemulgatoren in Lösung gehen und Kochsalz zurückbleibt. Nach Absaugen des Natriumchlorids und eines in Methanol unlöslichen geringfügigen Anteils (ca. 125 g getrockneter Filterrückstand, Cl-Gehalt ca. 50%) wird das methanolische Filtrat am Rotationsverdampfer bei ca. 30°C im Vakuum eingeengt. Es hinterbleibt ein gelbes, wachsartiges Substanzgemisch. Ausbeute: 440 g (Azoemulgator A).

Dünnschichtchromatographisch (Chloroform/Methanol-Gemisch 65:35) wurde nachgewiesen, dass im Azoemulgatorgemisch das aus Azodiisobuttersäureamidin in Gegenwart von Wasser rasch entstehende Azodiisobuttersäureamid sowie die durch Verseifung der sulfochlorierten Paraffine entstehenden Disulfonate nur in Spuren vorhanden sind.

Das IR-Spektrum des wasserfreien, festen Azoemulgators A, gemessen als KBr-Pressling, zeigt die für die Sulfonatgruppen charakteristische ν (S=O)-Banden bei 1190 und 1050 $cm^{-1}$. Im Carbonylgebiet treten Absorptionsbanden bei 1680 (Schulter) und 1640 sowie 1550 $cm^{-1}$ auf, die auf die verknüpfenden Strukturelemente

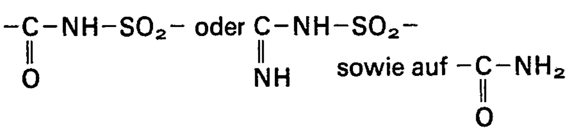

Struktureinheiten als nicht verknüpfende Elemente hinweisen.

Als mittleres Molekulargewicht (Zahlenmittelwert) wurde membranosmometrisch ein Wert von kleiner als 2,5 × $10^3$ ermittelt. Nach gelchromatographischen Untersuchung liegt das mittlere MG bei etwa $10^3$.

Der Azoemulgator A lässt sich analog auch aus dem Azodiisobuttersäureamidindihydrochlorid herstellen, wobei zur Bindung der Salzsäure eine entsprechend höhere Menge an Natronlauge verwendet wird (pro Mol Azoverbindung 2 mol NaOH mehr als oben angegeben).

*Beispiel 2:*

*(Polymerisationsversuche mit Azoemulgator A [Tabelle Ia, b, c] sowie Vergleichsversuche c, d und e)*

Serienpolymerisationsversuche wurden in 500 ml fassenden verkorkten Glasflaschen mit zusätzlichem Kronkorkenverschluss (vgl. Houben-Weyl, „Methoden der Organischen Chemie", Bd. 14/1, S. 147 [1961]) unter Ausschluss von Luftsauerstoff durchgeführt. Die zum Splitterschutz in Edelstahlkörbe eingesetzten Glasflaschen rotierten bei einer Drehzahl von 25 tr/min. Die Temperatur des Wasserbades wurde konstant gehalten. Die Korken der Flaschen wurden vor ihrer Entnahme aus den Stahlkörben mittels einer Stahlkanüle durchbohrt, um einen vorhandenen Überdruck zu entspannen.

In Tabelle I wird die Wirkungsweise des gemäss Beispiel 1 hergestellten Azoemulgators A verglichen mit der eines Gemisches bestehend aus Azodiisobuttersäureamidin als Initiator und einer dem Azoemulgator entsprechenden Menge eines handelsüblichen sekundären Alkanmonosulfonats auf Basis unverzweigter Paraffine mit durchschnittlich 15 C-Atomen.

Der Koagulatanteil der Dispersionen liegt bei Verwendung des Azoemulgators A nur geringfügig höher als beim Vergleichsversuch. Der Feststoffgehalt mit Azoemulgator A ist jedoch deutlich höher. Bemerkenswert an den erfindungsgemäss hergestellten Dispersionen sind insbesondere ihre geringere elektrische Leitfähigkeit sowie ihre geringere Tendenz zur Schaumbildung. Die Oberflächenspannung, welche umgekehrt proportional der mit einem Stalagmometer (1 ml Flüssigkeitsvolumen) gemessenen Tropfenzahl (die Tropfenzahl ist ein Mass für die Oberflächenspannung und ist definiert als die Zahl an Tropfen, in die 1 $cm^3$ Lösung oder Latex bei Zimmertemperatur [22° C] zerlegt wird) ist, liegt selbst bei Verwendung grösserer Mengen an Azoemulgator noch niedrig, dagegen fällt sie bei Verwendung grösserer Mengen an konventionellen Tensiden deutlich ab (vgl. Versuch c mit f in Tabelle I, Zeile 12 „Tropfenzahl Latex").

In der Grösse der Latexteilchen besteht (vgl. letzte Zeile der Tabelle I) kein signifikanter Unterschied, wenn entsprechende Mengen an Azoemulgator oder an Alkalialkanmonosulfonat verwendet werden. Eine 10%ige wässerige Lösung des verwendeten Azoemulgators A weist eine elektrische Leitfähigkeit von ca. 14 mS auf und eine entsprechende 10%ige Lösung des Alkalialkanmonosulfonats zeigt in Wasser eine Leitfähigkeit von etwa 14,4 mS. Unterscheiden sich demnach die eingesetzten Emulgatorlösungen in ihrer elektrischen Leitfähigkeit nur geringfügig, so besteht ein deutlicher Unterschied in der elektrischen Leitfähigkeit der mit Hilfe der verschiedenen Emulgatorsysteme hergestellten Polymerisatdispersionen.

Das verwendete Leitfähigkeitsmessgerät zeigte die Leitfähigkeit von Kaliumchloridlösungen bei Zimmertemperatur wie folgt an:

| | c (mol/l) KCl | Leitfähigkeit (mS) |
|---|---|---|
| 0,0745 g KCl/l | $10^{-3}$ | 0,12 |
| 0,746 g KCl/l | $10^{-2}$ | 1,3 |
| 7,456 g KCl/l | $10^{-1}$ | 11,2 |

Tabelle I

| | Erfindung | | | Vergleich | | |
|---|---|---|---|---|---|---|
| | a | b | c | d | e | f |
| Entionisiertes Wasser (g) | 163,6 | 152,8 | 109,6 | 159,1 | 148,3 | 105,1 |
| Styrol, destilliert (g) | 100 | 100 | 100 | 100 | 100 | 100 |
| Azoemulgator A 10%ig in Wasser (g) | 36,0 | 48,0 | 96,0 | — | — | — |
| Alkalialkanmonosulfonat mit ca. 15 C-Atomen, 10%ig in Wasser (Na-Salze) (g) | — | — | — | 36,0 | 48,0 | 96,0 |
| Azodiisobuttersäureamidin, 10%ig in Wasser (g) | — | — | — | 5,0 | 5,0 | 5,0 |
| Polymerisationsdauer (h) | 7 | 7 | 7 | 7 | 7 | 7 |
| Polymerisationstemperatur (°C) | 60 | 60 | 60 | 60 | 60 | 60 |
| Charakterisierung der Dispersionen | | | | | | |
| Koagulatgehalt (g) | 0,8 | 0,8 | 0,9 | 0,6 | 0,5 | 0,2 |
| Feststoffgehalt (%) | 32,9 | 34,0 | 34,8 | 31,9 | 33,8 | 30,8 |
| Elektrische Leitfähigkeit (mS) | 1,6 | 2,1 | 3,6 | 2,5 | 2,9 | 4,6 |
| Schaumrückbildung nach 30 s kräftigem Schütteln (s) | 100 | 130 | 180 | >300 | >300 | >300 |
| *Tropfenzahl Latex (Stalagmometer)* | 32 | 33 | 31 | 30 | 30 | 60 |
| Tropfenzahl Wasser (Stalagmometer) | 30 | 30 | 30 | 30 | 30 | 30 |
| Latexteilchendurchmesser (nm) nach Laserkorrelationsspektroskopie (LKS) | 113 | 107 | 94 | 108 | 107 | 95 |

*Beispiel 3:*

*(Gemäss Erfindung a bis f und Vergleiche g bis r)*

Bei gleicher Arbeitsweise, wie in Beispiel 2 geschildert, wird ein n-Butylacrylat/Styrol-Monomerengemisch in Emulsion erfindungsgemäss in Gegenwart von Azoemulgator A copolymerisiert (Tabelle II, Versuche a bis f).

Die Versuchsserie a bis f (Tabelle II) wird verglichen mit entsprechenden Versuchen, bei welchen als Emulgator ein Natriumalkanmonosulfonatgemisch und als Initiator Azodiisobuttersäureamidin eingesetzt und wobei das Gewichtsverhältnis Emulgator/Initiator, d.h. das Gewichtsverhältnis sek.-Alkanmonosulfonat/Azodiisobuttersäureamidin konstant gehalten wird (Tabelle II, Versuche g bis l).

Bei den Versuchen m bis r inTabelle II wurde anstelle des sekundären Alkanmonosulfonats ein kochsalzfreies Alkandisulfonat eingesetzt, welches durch Verseifung des in Beispiel 1 angeführten Gemisches sulfochlorierter Paraffine mit wässeriger Natronlauge hergestellt wurde.

Die Vergleichsversuche zeigen, dass die erfindungsgemäss hergestellten Dispersionen eine geringere Tendenz zur Schaumbildung und eine geringere elektrische Leitfähigkeit aufweisen.

In der Latexteilchengrösse entspricht die Versuchsserie a bis f, Tabelle II, weitgehend der Versuchsserie g bis l, Tabelle II. Dagegen sind die in Gegenwart des Alkalidisulfonatemulgators hergestellten Dispersionen wesentlich grobteiliger. Dieser Befund steht in Einklang mit der Tatsache, dass (vgl. Ausführungen Beispiel 1) im Azoemulgator A die durch Verseifung der sulfochlorierten Paraffine entstehenden Disulfonate nur in Spuren vorhanden sind.

Tabelle II

| | Erfindung | | | | | |
|---|---|---|---|---|---|---|
| | a | b | c | d | e | f |
| Entionisiertes Wasser (Tle) | 191,7 | 185,2 | 174,4 | 163,6 | 152,8 | 109,6 |
| Acrylsäure-n-butylester (Tle) | 57,3 | 57,3 | 57,3 | 57,3 | 57,3 | 57,3 |
| Styrol (Tle) | 42,7 | 42,7 | 42,7 | 42,7 | 42,7 | 42,7 |
| Azoemulgator A, 10%ig in Wasser (Tle) | 4,8 | 12,0 | 24,0 | 36,0 | 48,0 | 96,0 |
| Alkanmonosulfonat (Na-Salze) mit ca. 15 C-Atomen, 10%ig in Wasser (Tle) | — | — | — | — | — | — |
| Alkandisulfonat (Na-Salze) mit ca. 15 C-Atomen, 10%ig in Wasser (Tle) | — | — | — | — | — | — |
| Azodiisobuttersäureamidin, 10%ig in Wasser (Tle) | — | — | — | — | — | — |
| Polymerisationstemperatur (°C) | 60 → | | | | | |

Tabelle II

| | Erfindung | | | | | |
|---|---|---|---|---|---|---|
| | a | b | c | d | e | f |
| Polymerisationszeit (h) | 7 → | | | | | |
| Ausfall (Tle) | 3,1 | 2,0 | 1,0 | 0,7 | 0,6 | 0,6 |
| Feststoffgehalt Latex (Gew.-%) | 28,3 | 31,0 | 33,1 | 34,1 | 34,6 | 35,2 |
| Schaumrückbildung nach 30 s kräftigem Schütteln (s) | 5 | 10 | 15 | 25 | 60 | 250 |
| Elektrische Leitfähigkeit (mS) | 0,38 | 0,88 | 2,21 | 2,33 | 2,80 | 4,80 |
| Teilchengrösse (nm) nach LKS | 167 | 132 | 115 | 100 | 97 | 79 |
| Tropfenzahl Wasser | 24 | 24 | 24 | 24 | 24 | 24 |
| Tropfenzahl Latex | 32 | 34 | 32 | 32 | 32 | 36 |

Tabelle II *(Fortsetzung 1)*

| | Vergleich | | | | | |
|---|---|---|---|---|---|---|
| | g | h | i | j | k | l |
| Entionisiertes Wasser (Tle) | 191,7 | 185,2 | 174,4 | 163,6 | 152,8 | 109,6 |
| Acrylsäure-n-butylester (Tle) | 57,3 | 57,3 | 57,3 | 57,3 | 57,3 | 57,3 |
| Styrol (Tle) | 42,7 | 42,7 | 42,7 | 42,7 | 42,7 | 42,7 |
| Azoemulgator A, 10%ig in Wasser (Tle) | — | — | — | — | — | — |
| Alkanmonosulfonat (Na-Salze) mit ca. 15 C-Atomen, 10%ig in Wasser (Tle) | 3,8 | 9,5 | 19,0 | 28,5 | 38,0 | 76,0 |
| Alkandisulfonat (Na-Salze) mit ca. 15 C-Atomen, 10%ig in Wasser (Tle) | — | — | — | — | — | — |
| Azodiisobuttersäureamidin, 10%ig in Wasser (Tle) | 1,0 | 2,0 | 5,0 | 7,5 | 10,0 | 20,0 |
| Polymerisationstemperatur (°C) | 60 → | | | | | |
| Polymerisationszeit (h) | 7 → | | | | | |
| Ausfall (Tle) | 3,4 | 2,2 | 0,7 | 0,5 | 0,25 | 0,1 |
| Feststoffgehalt Latex (Gew.-%) | 31,7 | 33,2 | 33,7 | 33,8 | 34,8 | 34,6 |
| Schaumrückbildung nach 30 s kräftigem Schütteln (s) | 10 | 15 | 35 | >300 | >300 | >300 |
| Elektrische Leitfähigkeit (mS) | 0,66 | 1,45 | 2,52 | 3,50 | 4,50 | 7,80 |
| Teilchengrösse (nm) nach LKS | 166 | 115 | 100 | 94 | 90 | 79 |
| Tropfenzahl Wasser | 24 | 24 | 24 | 24 | 24 | 24 |
| Tropfenzahl Latex | 28 | 30 | 34 | 36 | 40 | 49 |

Tabelle II *(Fortsetzung 2)*

| | Vergleich | | | | | |
|---|---|---|---|---|---|---|
| | m | n | o | p | q | r |
| Entionisiertes Wasser (Tle) | 191,7 | 185,2 | 174,4 | 163,6 | 152,8 | 109,6 |
| Acrylsäure-n-butylester (Tle) | 57,3 | 57,3 | 57,3 | 57,3 | 57,3 | 57,3 |
| Styrol (Tle) | 42,7 | 42,7 | 42,7 | 42,7 | 42,7 | 42,7 |
| Azoemulgator A, 10%ig in Wasser (Tle) | — | — | — | — | — | — |
| Alkanmonosulfonat (Na-Salze) mit ca. 15 C-Atomen, 10%ig in Wasser (Tle) | — | — | — | — | — | — |
| Alkandisulfonat (Na-Salze) mit ca. 15 C-Atomen, 10%ig in Wasser (Tle) | 3,8 | 9,5 | 19,0 | 28,5 | 38,0 | 76,0 |
| Azodiisobuttersäureamidin, 10%ig in Wasser (Tle) | 1,0 | 2,5 | 5,0 | 7,5 | 10,0 | 20,0 |
| Polymerisationstemperatur (°C) | 60 → | | | | | |
| Polymerisationszeit (h) | 7 → | | | | | |
| Ausfall (Tle) | 3,1 | 2,5 | 1,5 | 1,2 | 1,1 | 0,5 |
| Feststoffgehalt Latex (Gew.-%) | 27,6 | 30,1 | 31,3 | 34,5 | 31,2 | 31,4 |
| Schaumrückbildung nach 30 s kräftigem Schütteln (s) | 30 | 30 | 70 | <300 | <300 | <300 |

Tabelle II *(Fortsetzung 2)*

| | Vergleich | | | | | |
|---|---|---|---|---|---|---|
| | m | n | o | p | q | r |
| Elektrische Leitfähigkeit (mS) | 0,85 | 2,0 | 3,5 | 4,8 | 6,8 | 10,8 |
| Teilchengrösse (nm) nach LKS | 240 | 185 | 153 | 153 | 150 | 220 |
| Tropfenzahl Wasser | 24 | 24 | 24 | 24 | 24 | 24 |
| Tropfenzahl Latex | 32 | 33 | 34 | 33 | 36 | 42 |

*Beispiel 4:*

*(Vergleichsbeispiele)*

Nachfolgend angeführte Verbindungen werden ohne Zuhilfenahme von Emulgatoren zur Polymerisation eines Acrylsäure-n-butylester/Styrol-Gemisches eingesetzt

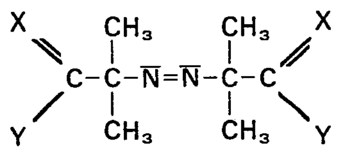

a) X = NH Y = $-NH_2$
b) X = NH Y = $-NH_3^+Cl^-$
c) X = NH Y = $-O-C_2H_5$
d) X = $N-CH_2-CH_2-OH$
Y = $-NH-CH_2-CH_2-OH$
e) X = $N-CH_2-CH(OH)-CH_3$
Y = $-NH-CH_2-CH(OH)-CH_3$
f) X = $N-CH_2-CH(OH)-CH_3$ Y = $-NH_2$

Ferner wird noch die Verbindung

g) 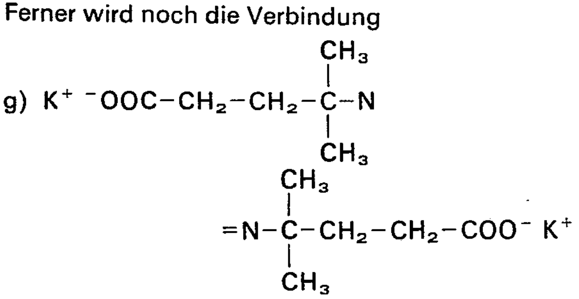

als Azoemulgator getestet.

Die Versuche wurden analog der in Beispiel 3, Tabelle II, a bis f, angeführten Versuchsreihe unter gleichen Bedingungen (60°C, 7 h) durchgeführt. Es wurden gleiche Gewichtsmengen der Verbindungen a bis g eingesetzt. Die Auswertung der ingesamt 42 Versuche ergab, dass in keinem Fall ein Latex erhalten werden konnte. Vielmehr entstand bei Verwendung der Verbindung:

a) zähflüssiger Brei mit hohem Monomerenanteil
b) Koagulat
c) zähflüssiger Brei mit hohem Monomerenanteil
d) Brei und Koagulat
e) kein Polymerisat
f) kein Polymerisat oder Koagulat
g) kein Polymerisat

*Beispiel 5*

*(Azoemulgator B [erfindungsgemäss])*

Zu 109,2 g (0,27 mol) eines Gemisches sulfochlorierter Paraffine (mittlere C-Zahl 15, Sulfochlorgehalt 17%, entsprechend 2 Sulfochlorgruppen pro C-15-Alkan und zusätzlichen 3 bis 4,5% C-Kettenchlor) werden unter Rühren und Aussenkühlung mit Eiskochsalzmischung (ca. −15°C Aussentemperatur) 50 g (0,134 mol) Azodiisobuttersäure-(N,N'-bishydroxiethyl)amidin (M = 374)

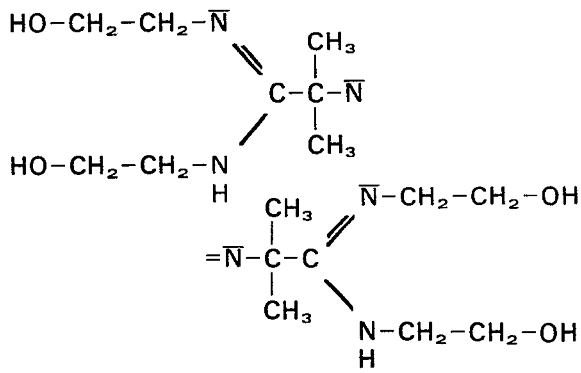

sowie eine Lösung von 32,1 g (0,8 mol) NaOH in 100 g destilliertem Wasser innerhalb des pH-Bereiches von 7 bis 10 gleichzeitig so zugegeben, dass die Innentemperatur nicht über +30°C steigt.

Nach beendeter Reaktion besitzt das Reaktionsgemisch einen pH-Wert von 8 und ist durchscheinend und honigbraun. Das Wasser wird schonend am Rotationsverdampfer im Vakuum entfernt, was ohne Schaumbildung möglich ist.

Der Rückstand wird mit Ethanol (ca. 700 ml) verrührt, wobei Kochsalz in grobkristalliner, gut filtrierbarer Form anfällt (30 g) und der Azoemulgator in Lösung geht.

Nach Entfernen des Alkohols und Trocknen des Rückstandes (60 min, 30°C, ca. 0,5 mbar) hinterbleiben 125 g einer gut wasserlöslichen Paste.

Ein Teil wird in der Trockenpistole schonend bei 30°C über Phosphorpentoxid getrocknet (5 h).

Elementaranalytische Zusammensetzung des Substanzgemisches (%): C: 47,5; H: 8,3; N: 7,1; O: 19,4; S: 10,5; Cl: 3,2. Rest $Na^{\oplus}$.

Da es sich um ein Substanzgemisch handelt, kann keine eindeutige Strukturformel angegeben werden.

Als mittleres Molekulargewicht (Zahlenmittelwert) wurde membranosmometrisch ein Wert von kleiner als $2,5 \times 10^3$ ermittelt.

Nach gelchromatographischen Untersuchungen liegt das mittlere Molekulargewicht bei ca. $10^3$.

Die verdünnte wässerige Lösung des Azoemulgators B schäumt und besitzt in einer Konzentration von 10 Gew.-% eine elektrische Leitfähigkeit von 13,8 mS. Eine Lösung von 7,46 g KCl in 1 l Wasser (0,1 mol/l) besitzt eine Leitfähigkeit von 12 mS, mit gleichem Gerät gemessen.

Die Tropfenzahl von 50 g Wasser wird durch Zusatz von 10%iger Azoemulgator-B-Lösung wie folgt erhöht

| Zusatz von Azoemulgator B von einer 10%igen Lösung (ml) | Tropfenzahl von 1 ml wässeriger Lösung, nach Zusatz von B zu 50 g Wasser |
|---|---|
| 0 (reines Wasser) | 25 |
| 0,2 | 27 |
| 0,4 | 32 |
| 0,6 | 38 |
| 0,8 | 43 |
| 1,0 | 47 |
| 1,2 | 49 |
| 1,4 | 50 |
| 1,6 | 52 |
| 1,8 | 54 |
| 2,2 | 54 |
| 2,8 | 56 |
| 3,6 | 57 |
| 5,2 | 58 |
| 8,0 | 58 |

Azoemulgator B zeigt demnach typische Tensideigenschaften.

*Beispiel 6:*

*(Polymerisationsversuche mit Azoemulgator B)*

Gemäss Beispiel 2 werden die in Tabelle III angeführten Polymerisationsversuche durchgeführt.

Auch mit Hilfe des Azoemulgators B ist es demnach möglich, feinteilige und wenig schäumende Dispersionen herzustellen.

Tabelle III

| Versuch | IIIa | IIIb | IIIc | IIId |
|---|---|---|---|---|
| Entionisiertes Wasser (Tle) | 152,8 | 163,60 | 152,80 | 109,60 |
| Styrol (Tle) | 100 | 42,70 | 42,70 | 42,70 |
| Acrylsäure-n-butylester (Tle) | 0,0 | 57,30 | 57,30 | 57,30 |
| Azoemulgator B gemäss Beispiel 5, 10%ig in $H_2O$ (Tle) | 48,0 | 36,0 | 48,0 | 96,0 |
| Polymerisationstemperatur (°C) | 60 | 60 | 60 | 60 |
| Polymerisationsdauer (h) | 7 | 7 | 7 | 7 |
| Feststoffgehalt Latex (%) | 33,5 | 32,4 | 33,1 | 34,2 |
| Ausfall (Tle) | 3,7 | 3,2 | 2,2 | 3,3 |
| Teilchendurchmesser (nm) nach LKS (Latexteilchen) | 122 | 125 | 115 | 145 |
| Leitfähigkeit (mS) | 2,5 | 1,7 | 2,6 | 4,9 |
| Tropfenzahl Latex | 26,0 | 35 | 40 | 55 |
| Tropfenzahl Wasser | 25 | 25 | 25 | 25 |
| Schaumrückbildung (s) | 60 | 35 | 70 | 250 |

*Beispiel 7:*

*(Polybutadienlatex mit Azoemulgator A)*

In einen 6-l-Autoklav werden unter Stickstoffatmosphäre eingefüllt

| | |
|---|---|
| Entionisiertes Wasser | 1640 g |
| Butadien | 1000 g |

Das Gemisch wird auf 60°C aufgeheizt. Danach werden 360 ml einer 10%igen Lösung des Azoemulgators A (gemäss Beispiel 1) aus einem kleinen Druckbehälter in den 6-l-Autoklaven eingedrückt.

Nach einer Polymerisationzeit von 7½ h werden ca. 3 kg Latex mit einem Feststoffgehalt von 32,0 Gew.-% erhalten. Der Feststoffgehalt während der Polymerisation gezogener Proben beträgt nach Zugabe des Azoemulgators nach der

1. Stunde: 1,0 Gew.-%
2. Stunde: 4,0 Gew.-%
3. Stunde: 7,0 Gew.-%
4. Stunde: 14,5 Gew.-%
5. Stunde: 20,5 Gew.-%
6. Stunde: 28,5 Gew.-%
7. Stunde: 30,5 Gew.-%

Der Druck fällt während der Polymerisation von 12,8 bar auf 7 bar ab (bei 60°C).

Der Koagulatanteil des Latex beträgt ca. 0,5%, bezogen auf Polymerisat, die Latexteilchendurchmesser besitzen eine Grösse von 85 nm, der Latex ist dünnflüssig und schäumt nicht, und die elektrische Leitfähigkeit des Latex liegt bei 1,7 mS.

Das Polymerisat ist weitgehend vernetzt und daher nur zum Teil (60%) löslich.

Wird der Anteil an Azoinitiator verringert und gleichzeitig ein üblicher Emulgator wie z.B. Kaliumoleat sowie ein Regler wie Dodecylmercaptan mitverwendet, so kann der Gelanteil herabgesetzt werden.

*Beispiel 8:*

*(PVC-Dispersion mit Azoemulgator A)*

In einen 6-l-Autoklav werden unter Stickstoffatmosphäre eingefüllt

Entionisiertes Wasser 1640 g
Vinylchlorid 1000 g

Das Gemisch wird auf 60°C aufgeheizt.

Wenn 60°C erreicht sind, werden 360 ml einer 10%igen Lösung des Azoemulgators A (gemäss Beispiel 1) aus einem kleinen Druckbehälter in den 6-l-Autoklaven eingedrückt.

Nach einer Polymerisationzeit von 7 h wird eine grobdisperse Dispersion mit einer Teilchengrösse von ca. 500 bis 2000 nm mit einem Feststoffgehalt von ca. 33% erhalten.

Das Polymerisat ist löslich in Tetrahydrofuran und besitzt eine Viskositätszahl von $[\eta] = 0,8$ (bei 25°C).

## Patentansprüche

1. Oberflächenaktive Azoverbindungen, erhältlich durch Umsetzung von Azoverbindungen der allgemeinen Formel (I)

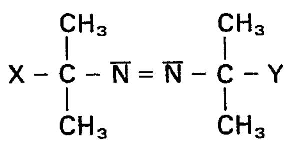

(I)

worin X und Y gleich oder verschieden

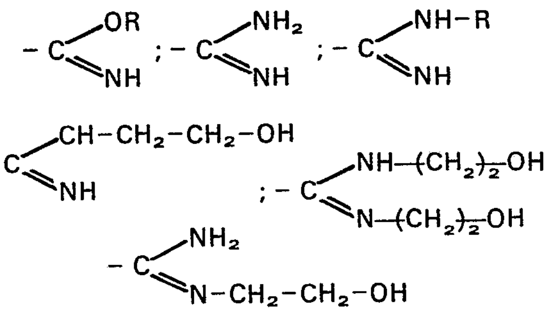

und R = $C_1$ bis $C_4$-Alkyl bedeuten, mit mindestens einem $C_{10}$ bis $C_{18}$-Paraffindisulfonsäuredihalogenid unter Halogenwasserstoffabspaltung in wässerigem Medium oder einem gegenüber den Reaktanten unter den Prozessbedingungen inerten Lösungsmittel oder Lösungsmittelgemisch, worin die Reaktanten zumindest teilweise gelöst sind, bei Temperaturen von 0 bis 30°C in Gegenwart einer dem verseifbaren Halogen im wesentlichen äquivalenten Menge einer Base.

2. Oberflächenaktive Azoverbindungen gemäss Anspruch 1, dadurch gekennzeichnet, dass auf 1 mol der Azoverbindung der Formel (I) 1 bis 2 mol des Paraffindisulfonsäuredihalogenids eingesetzt werden.

3. Oberflächenaktive Azoverbindungen gemäss Anspruch 1, dadurch gekennzeichnet, dass als Base Alkalimetallhydroxide oder Ammoniak eingesetzt werden.

4. Verwendung der oberflächenaktiven Azoverbindungen gemäss Anspruch 1 bei der Emulsions- und Dispersionspolymerisation eines oder mehrerer olefinisch ungesättigter Monomerer als Initiator und Emulgator.

## Claims

1. Surface-active azo compounds, obtainable by reacting azo compounds of the general Formula (I)

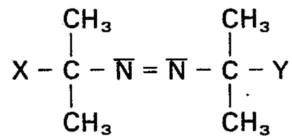

(I)

wherein X and Y, which are identical or different, denote

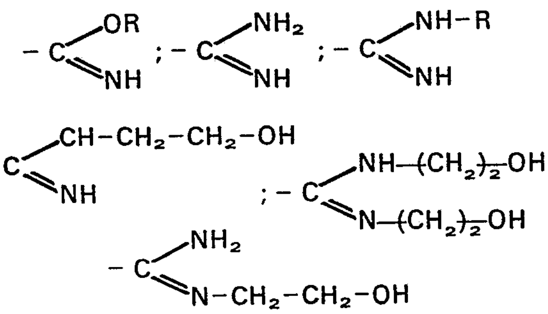

and R denotes $C_1$ to $C_4$-alkyl, with at least one $C_{10}$ to $C_{18}$ paraffin disulphonic acid dihalide, with dehydrohalogenation, in an aqueous medium or a solvent or solvent mixture which is inert towards the reactants under the process conditions and in which the reactants are at least partly dissolved, at temperatures of 0 to 30°C and in the presence of a base in a quantity which is substantially equivalent to the hydrolysable halogen.

2. Surface-active azo compounds according to Claim 1, characterised in that 1 to 2 mol of the paraffin disulphonic acid dihalide are used per mole of the azo compounds of the Formula (I).

3. Surface-active azo compounds according to Claim 1, characterised in that alkali metal hydroxides or ammonia are used as the base.

4. Use of the surface-active azo compounds according to Claim 1 as the initiator and emulsifier in the emulsion and dispersion polymerisation of one or more olefinically unsaturated monomers.

## Revendications

1. Composés azoïques tensio-actifs pouvant être obtenus par réaction de composés azoïques de formule générale (I)

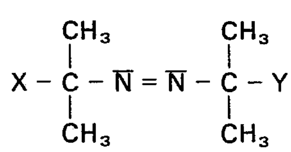

(I)

dans laquelle X et Y, égaux ou différents, représentent des restes

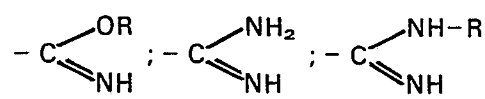

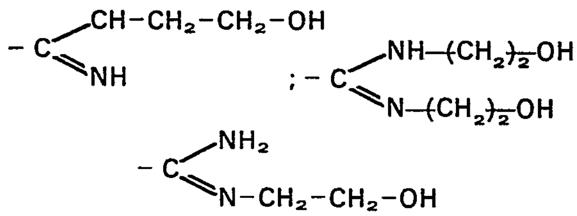

;

et R est un reste alkyle en $C_1$ à $C_4$, avec au moins un dihalogénure d'un acide (paraffine en $C_{10}$ à $C_{18}$)-disulfonique, avec élimination d'halogénure d'hydrogène, en milieu aqueux ou dans un solvant ou un mélange de solvants inertes vis-à-vis des corps réactionnels dans les conditions opératoires, où les corps réactionnels sont solubles, tout au moins partiellement, à des températures de 0 à 30°C en présence d'une quantité d'une base équivalant principalement à l'halogène saponifiable.

2. Composés azoïques tensio-actifs suivant la revendication 1, caractérisés en ce qu'on utilise par mole du composé azoïque de formule (I) 1 à 2 mol du dihalogénure d'acide paraffinedisulfonique.

3. Composés azoïques tensio-actifs suivant la revendication 1, caractérisés en ce qu'on utilise comme base des hydroxydes de métaux alcalins ou l'ammoniac.

4. Utilisation des composés azoïques tensio-actifs suivant la revendication 1 dans la polymérisation en émulsion et en dispersion d'un ou de plusieurs monomères à insaturation oléfinique comme initiateurs et émulsionnants.